# EUROPEAN PATENT SPECIFICATION

(11) **EP 2 674 492 B1**
(45) Date of publication and mention of the grant of the patent: **04.04.2018**
(21) Application number: 12744619.3
(22) Date of filing: 09.02.2012
(51) Int. Cl.: C12N 15/09, C12M 1/00, C12Q 1/68, G01N 37/00

(54) **METHOD FOR DETECTING NUCLEIC ACID**
VERFAHREN ZUM NACHWEIS VON NUKLEINSÄUREN
MÉTHODE DE DÉTECTION D'UN ACIDE NUCLÉIQUE

(30) Priority: 10.02.2011 JP 2011027588
(43) Date of publication of application: 18.12.2013
(73) Proprietor: Mitsubishi Chemical Corporation, Tokyo 100-8251 (JP)
(72) Inventor: TOGAWA, Naoyuki, Tokyo 100-8251 (JP)
(74) Representative: Hoffmann Eitle
(86) International application number: PCT/JP2012/052968
(87) International publication number: WO 2012/108499

(56) References cited:
- EP-A1- 1 491 888
- WO-A1-01/06012
- JP-A- 2008 128 670
- JP-A- 2009 537 126
- ALEXEY ATRAZHEV ET AL: "In-gel technology for PCR genotyping and pathogen detection", ANALYTICAL CHEMISTRY, AMERICAN CHEMICAL SOCIETY, US, [Online] vol. 82, no. 19, 1 October 2010 (2010-10-01), pages 8079-8087, XP002729512, ISSN: 0003-2700, DOI: 10.1021/AC1013157 Retrieved from the Internet: URL:http://pubs.acs.org/doi/abs/10.1021/ac 1013157> [retrieved on 2010-09-01]
- KOLCHINSKY A. ET AL.: 'Analysis of SNPs and other genomic variations using gel-based chips.' HUM. MUTAT. vol. 19, no. 4, 2002, pages 343 - 360, XP008164154

## Description

### TECHNICAL FIELD

The present invention relates to a method for detecting a nucleic acid using a DNA microarray, etc. Specifically, the present invention relates to: a method for specifically detecting a nucleic acid by means of the on-chip PCR reaction utilizing a gel-held type DNA microarray utilizing the molecular sieving effect of the spot gel; and the like.

### BACKGROUND ART

The DNA microarray is a tool for performing a hybridization reaction with a sample nucleic acid using an immobilized DNA as a probe.

Recently, practical use of such a DNA microarray for detection of a specific nucleotide sequence in a nucleic acid as a specimen has been being promoted in the fields of search of disease-associated genes, clinical diagnosis, etc.

As DNA microarrays, for example, a DNA microarray in which probes are sequentially synthesized on a two-dimensional surface using photolithography (see Patent Document 1); a DNA microarray in which probes synthesized in advance are spotted on a two-dimensional surface (see Patent Document 2); a DNA microarray in which a plurality of grooves or through-holes are formed in a substrate such as a resin plate and a DNA-containing gel is held in each of them (see Patent Document 3); a DNA microarray in which spots of gel containing DNA, etc. are arranged on a flat base (see Patent Document 4); and the like are known. A part of the present inventors also have developed a DNA microarray obtained by preparing a hollow fiber-arranged body in which gel is held in the hollow portion of each hollow fiber and slicing the body in the direction crossing the fiber axis of the body (see Patent Document 5).

Since the amount of a target nucleic acid existing in a specimen is usually extremely small, in a conventional method for using a DNA microarray, it is required to amplify a target nucleic acid by means of the T7 amplification method, the PCR method or the like in advance. Further, there is a case where a hybridization reaction must be performed overnight. Therefore, it was thought that processes from reaction to detection must be more simplified.

In order to reduce the time that elapses before a target nucleic acid is detected, many techniques for performing a PCR reaction on a DNA microarray have been disclosed. For example, Non-Patent Document 1 discloses a technique of performing DNA amplification by means of solid phase PCR (Polymerase Chain Reaction) using a DNA microarray in which a DNA strand as a primer is covalently-bonded to the surface of a glass substrate modified using a given amino-silane reagent.

Further, in Non-Patent Document 2, a DNA microarray in which poly(methyl methacrylate) is used instead of a glass substrate and a DNA fragment is immobilized on the surface thereof was used to evaluate hybrid properties with a given DNA strand and thermal stability under PCR-like environment.

As examples of more specific applications, a rapid microorganism identification technique and a single nucleotide polymorphism detection technique have been attempted to be developed as shown in Non-Patent Documents 3 and 4.

However, these techniques still have several problems. Particularly, as typical problems of PCR reaction on a DNA microarray in which a nucleic acid is immobilized on a substrate, the following points 1) to 3) are mentioned:
1) an immobilized primer is dropped off at the time of a heat cycle (at the time of PCR reaction);
2) when performing PCR reaction on the surface of a substrate, the efficiency of extension reaction of a nucleic acid is low; and
3) at the time of PCR reaction, non-specific detection may occur.

Regarding these problems, several solutions have been considered. However, for example, a special chemical modification on a substrate is required and a usual nucleotide cannot be used (a substituted nucleotide is used), and thus, the practicability thereof is low (see Patent Documents 6-9).

Further, aside from these, the DNA microarray technique in which a nucleic acid is not immobilized on the surface of a substrate and a gel pad is utilized has been developed. It is known that it enables an amplification reaction and a detection reaction by extension of an individual base in the gel pad on the DNA microarray (see Non-Patent Document 5).

In the case of a gel-used DNA microarray (see Patent Documents 3-5), the amount of a probe which can be immobilized on one section is larger compared to a DNA microarray in which a probe is immobilized on a two-dimensional surface. Therefore, it can be said that it is a DNA microarray having an excellent hybridization efficiency (the ratio of the nucleic acid bound to the probe to the nucleic acid subjected to hybridization).

In the case of the above-described gel-utilized DNA microarray, high hybridization efficiency is attained when a sample to be tested (hereinafter referred to as "specimen") is sufficiently diffused in the porous structure of gel and reacted with the probe in the gel. However, when using already-known gel DNA microarrays, specimens cannot be sufficiently diffused in the porous structure of gel, and in some cases, only the surface of gel is used for testing.

For the purpose of promoting diffusion of specimens by increasing the effective pore size of the porous structure of gel and the like, the type and concentration of a monomer constituting a gel have been examined (see Patent Document 10 and Non-Patent Documents 6-9). For example, Non-Patent Document 6 describes a method of using 8% by mass of acrylamide gel, and Non-Patent Documents 7 and 8 describe that DNAs having up to 500 base lengths, were successfully subjected to hybridization by using 5% by mass of methacrylamide gel. Further, Patent Document 10 describes that high hybridization efficiency can be obtained by using a gel containing 2 to 7% by mass of N,N-dimethylacrylamide as a gel suitable for use in a DNA microarray. Moreover, a method of utilizing a thermosensitive gel such as N-isopropylacrylamide is also known (see Non-Patent Document 9).

Non-Patent Document 10 describes an in-gel technology for PCR genotyping and pathogen detection.

Patent Document 11 features derivative nucleic acids and uses thereof.

### PRIOR ART DOCUMENTS

### PATENT DOCUMENTS

Patent Document 1: US Patent No. 5405783
Patent Document 2: US Patent No. 5601980
Patent Document 3: Japanese Laid-Open Patent Publication No. 2000-60554
Patent Document 4: US Patent No. 6682893
Patent Document 5: Japanese Laid-Open Patent Publication No. 2000-270877
Patent Document 6: Japanese Laid-Open Patent Publication No. 2006-230335
Patent Document 7: Japanese Laid-Open Patent Publication No. 2007-330104
Patent Document 8: Japanese Laid-Open Patent Publication No. 2007-222010
Patent Document 9: Japanese Laid-Open Patent Publication No. 2009-219358
Patent Document 10: Japanese Patent No. 3654894
Patent Document 11: WO 01/06012

### NON-PATENT DOCUMENTS

Non-Patent Document 1: Adessi, Celine et al, "Solid phase DNA amplification: Characterisation of primer attachment and amplification mechanisms", Nucleic Acids Res., 2000, Vol. 20, No. 20, e87
Non-Patent Document 2: Fixe, F. et al., "Functionalization of poly(methyl methacrylate) (PMMA) as a substrate for DNA microarrays", Nucleic Acids Res., Jan. 2004
Non-Patent Document 3: Georg, M. et al., "Microarray-Based Identification of Bacteria in Clinical Samples by Solid-Phase PCR Amplification of 23S Ribosomal DNA Sequences", JOURNAL OF CLINICAL MICROBIOLOGY, Mar. 2004, pp. 1048-1057
Non-Patent Document 4: Martin H. et al., "Detection of Single Base Alterations in Genomic DNA by Solid Phase Polymerase Chain Reaction on Oligonucleotide Microarrays", Analytical Biochemistry, 299, 24-30, 2001
Non-Patent Document 5: Svetlana D. et al., "Polymorphism analysis and gene detection by minisequencing on an array of gel-immobilized primers", Nucleic Acides Res., 1999, Vol. 27, No. 18, el9
Non-Patent Document 6: Gennady Yershov et al., "DNA analysis and diagnostics on oligonucleotide microchips", Proc. Natl. Acad. Sci. USA, Vol. 93, pp. 4913-4918, May 1996
Non-Patent Document 7: A. Yu. Rubina et al., "Hydrogel drop microchips with immobilized DNA: properties and methods for large-scale production", Analytical Biochemistry, Vol. 325, pp. 92-106, 2004
Non-Patent Document 8: Dmitry A. Khodakov et al., "An oligonucleotide microarray for multiplex realtime PCR identification of HIV-1, HBV, and HCV", BioTechniques, Vol. 44, No. 2, pp. 241-248, 2008
Non-Patent Document 9: Shuji Sakohara et al., the 69th Annual Meeting of Society of Chemical Engineers 1316: "Application of thermosensitive porous gel for construction of DNA chips"
Non-Patent Document 10: Alexey Atrazhev et al, Analytical Chemistry, vol. 82, no. 19, 1 October 2010, pages 8079-8087

### SUMMARY OF THE INVENTION

### PROBLEMS TO BE SOLVED BY THE INVENTION

Conventionally, a DNA microarray utilizing a gel in which the effective pore size of the porous structure of the gel is increased to improve diffusion of a specimen has a higher sensitivity and a higher thermal stability (a probe is not dissociated from a portion on which it is immobilized by heat) compared to a DNA microarray in which a probe is immobilized on a substrate. Particularly, it was useful as a DNA microarray for gene expression analysis.

However, in the case of gene expression analysis, an RNA amplified and purified in advance may be subjected to hybridization to the probe on the DNA microarray, but in the case of performing a PCR reaction on the DNA microarray, an unexpected reaction may occur in a liquid phase in which the array is actually immersed, on the surface of the array, in the inside of the array or the like. Thus, in terms of specificity, the same problem as that for the DNA microarray in which the probe is immobilized on the substrate exists. In particular, in order to simultaneously perform a PCR reaction and a hybridization reaction on an array, it is required to use a DNA microarray having a higher specificity.

### MEANS FOR SOLVING THE PROBLEMS

The present inventors diligently made researches and found that nucleic acid detection having a higher specificity can be performed by using a DNA microarray which utilizes both the molecular sieving effect of a gel and the specificity of a probe.

That is, the present inventors found that the specificity of the detection of a desired nucleic acid is improved by simultaneously performing a nucleic acid amplification reaction according to a nucleic acid amplification method such as PCR and a hybridization reaction on a DNA microarray utilizing a gel, and thus achieved the present invention.

The current invention is defined, *inter alia,* by the following items:
[1] A method for detecting a nucleic acid, which comprises:
   (a) a step wherein a gel, on which a nucleic acid probe is immobilized, is brought into contact with a reaction solution which contains a nucleic acid that serves as a template for nucleic acid amplification, a primer set for nucleic acid amplification, a nucleotide unit and a DNA extension enzyme;
   (b) a step wherein the gel and the reaction solution are subjected to a heat cycle for performing a nucleic acid amplification reaction;
   (c) a step wherein nucleic acid fragments having a specific base length are selected from among the amplified nucleic acid fragments, wherein the selecting of the nucleic acid fragments having the specific base length occurs by utilizing a molecular sieving effect of the probe-immobilized gels; and
   (d) a step wherein the selected nucleic acid fragments are detected;
   wherein the ratio (V/S) of the volume of the gel (V (µm³)) to the contact surface area of the gel on which the probe is immobilized and the reaction solution (S (µm²)) is 50 or more;
   wherein the concentration of the gel on which the probe is immobilized is more than 2% by mass and less than 5% by mass;
   wherein the gel on which the probe is immobilized comprises a substituted (meth)acrylamide derivative and/or an agarose derivative; and
   wherein the primer set is determined such that the nucleotide sequence of an oligonucleotide that serves as a probe is included in the middle of a sequence to be amplified.
[2] The method according to item 1, wherein the gel on which the probe is immobilized is held in a well or through-hole in a substrate.

The present description further provides:
(1) A method for detecting a nucleic acid, which comprises:
   (a) a step wherein a gel, on which a probe is immobilized, is brought into contact with a reaction solution which contains a nucleic acid that serves as a template for nucleic acid amplification, a primer set for nucleic acid amplification, a nucleotide unit and a DNA extension enzyme;
   (b) a step wherein the gel and the reaction solution are subjected to a heat cycle for performing a nucleic acid amplification reaction;
   (c) a step wherein nucleic acid fragments having a specific base length are selected from among the amplified nucleic acid fragments; and
   (d) a step wherein the selected nucleic acid fragments are detected.

   In the detection method, as the gel on which the probe is immobilized, for example, a plurality of types of gels with different gel concentrations can be used. Further, the gel may be held, for example, in a well or through-hole in a substrate, and may comprise a substituted (meth)acrylamide derivative and/or an agarose derivative. Moreover, the gel preferably has a gel concentration that is appropriately set according to the size (base length) of a nucleic acid as a detection target. For example, the gel concentration is more than 2 % by mass and less than 5 % by mass.
   Further, in the detection method, for example, the ratio of the volume of the gel (V (µm³)) to the contact surface area of the gel on which the probe is immobilized and the reaction solution (S (µm²)) (i.e., a value obtained by dividing a value of V by a value of S (V/S)) may be 50 or more.
(2) A microarray, by which a plurality of types of gels with different gel concentrations are carried, and wherein a probe is immobilized on the gels.
(3) A method for producing the microarray according to claim 7, which comprises:
   (a) a step wherein a plurality of hollow fibers are three-dimensionally arranged so that the fiber axial directions of the hollow fibers become the same, and wherein the arrangement is fixed with a resin to produce a hollow fiber bundle;
   (b) a step wherein a plurality of types of gel precursor solutions with different monomer concentrations containing a probe are introduced into the respective hollow portions of the hollow fibers of the hollow fiber bundle;
   (c) a step wherein the gel precursor solutions introduced into the hollow portions are reacted (copolymerization reaction) to hold a gel-like product comprising the probe (probe-immobilized gel) in the hollow portions of the hollow fibers; and
   (d) a step wherein the hollow fiber bundle is sliced in the direction crossing the longitudinal direction of the fibers into thin sections.

### ADVANTAGEOUS EFFECT OF THE INVENTION

According to the present invention, it is possible to provide: a method for detecting a nucleic acid, which efficiently eliminates non-specific detection of nucleic acids other than the nucleic acid as a detection target, so that detection specificity of the nucleic acid as a detection target can be further improved (in particular, the false-positive rate can be reduced) in a nucleic acid amplification reaction (PCR or the like) on a DNA microarray utilizing a gel; and a microarray to be used in the detection method. In the detection method, by suitably setting the gel concentration (the porous structure of the gel) according to the size (base length) of a nucleic acid to be amplified (nucleic acid as a detection target), the molecular sieving effect of the gel can be improved and the detection method having a higher specificity can be provided. The detection method and the like of the present invention are suitably applied to the treatment of a large amount of specimen and have excellent practicability and utility.

### BRIEF DESCRIPTION OF THE DRAWINGS

FIG. 1 shows a through-hole type DNA microarray mounted on a thin type 96-well plate made of saturated cyclic polyolefin.
FIG. 2 shows a state in which a thin type 96-well plate made of saturated cyclic polyolefin is set on a commercially-available thermal cycler (Life Technologies Corporation, GeneAmp 9700).
FIG. 3 is a schematic view of an amplification reaction (early stage of amplification reaction) of a DNA as a detection target in a reaction well (reaction solution).
FIG. 4 is a schematic view showing selective diffusion of amplified products into a spot gel of a through-hole type array.
FIG. 5 is a schematic view of a PCR reaction in a spot gel.
FIG. 6 is a schematic view showing an arrangement fixture for producing a hollow fiber bundle (hollow fiber-arranged body).
FIG. 7 is a schematic view showing the gel concentration of each spot and the position on which a probe is mounted in a DNA microarray.
FIG. 8 shows an electrophoretogram of amplified products with different sizes and a schematic view showing positions of these amplified products which can hybridize to the probe.
FIG. 9 shows results of examination of the molecular sieving effect regarding the amplified product of 123 bp.
FIG. 10 shows results of examination of the molecular sieving effect regarding the amplified product of 413 bp.
FIG 11 shows results of examination of the molecular sieving effect regarding the mixture of the amplified product of 827 bp and the amplified product of 1191 bp.
FIG. 12 is a schematic view showing non-selective diffusion of an amplified product in a spot on the flat plate-like array used in Comparative Example 1.

### BEST MODE FOR CARRYING OUT THE INVENTION

Hereinafter, the present invention will be described in detail. The scope of the present invention is not limited to the description. In addition to the following examples, the present invention can be suitably changed and then practiced within a range in which the effects of the present invention are not reduced.

The present description relates to a method for specifically detecting a nucleic acid by using a DNA microarray utilizing synergistic effects of the property (molecular sieving effect) of the spot gel and the probe specificity and by simultaneously performing a nucleic acid amplification reaction (PCR or the like) and a hybridization reaction on the DNA microarray. The spot gel is a gel obtained by copolymerizing a gel precursor comprising one or more substituted (meth)acrylamide derivatives or agarose derivatives, a cross-linking agent and a given probe.

As used herein, "DNA microarray" does not refer to a microarray on which only a DNA (deoxyribonucleic acid) is immobilized, but refers to a microarray on which a "probe" is immobilized. Further, "reaction on a microarray" means a "reaction in the entire reaction system including a microarray and a reaction solution", and does not particularly mean a reaction only on the surface of and in the inside of a gel in an array utilizing the gel.

Hereinafter, one embodiment of a method for producing a DNA microarray to be used in the present invention will be described.

### 1. Method for producing a DNA microarray utilizing a gel

### <Probc which is immobilized on gel>

As used herein, "probe" refers to nucleic acids such as deoxyribonucleic acid (DNA), ribonucleic acid (RNA) and peptide nucleic acid (PNA), and includes protein, lipid and the like in some cases. These probes can be obtained from commercially-available synthetic products, living cells or the like. For example, the extraction of DNA from living cells can be carried out according to the method of Blin et al. (Nucleic Acids Res. 3. 2303 (1976)) or the like, and the extraction of RNA can be carried out according to the method of Favaloro et al. (Methods. Enzymol. 65. 718 (1980)) or the like. Particularly in the present invention, an elongation reaction of a nucleic acid occurs on the surface of and in the inside of a gel in a DNA microarray, and therefore, the "probe" simultaneously has the function as a primer.

Further, the nucleic acid to be used as the probe may be in the form of a chain or ring and is not particularly limited. Examples thereof include plasmid DNA, chromosomal DNA, and RNA (in the case of a virus, etc.). It is also possible to use a DNA fragment chemically modified or cleaved by a restriction enzyme, a DNA synthesized by an enzyme or the like in a test tube, a chemically synthesized oligonucleotide and the like.

As described below, the probe is immobilized to the net-like structure of the gel by a copolymerization reaction with a substituted (meth)acrylamide derivative or an agarose derivative and a cross-linking agent. Therefore, it is preferred that a copolymerizable unsaturated functional group has been introduced into the probe (hereinafter referred to as "the modified probe"). Examples of the unsaturated functional group include a (meth)acrylamide group and a glycidyl group. The unsaturated functional group may be introduced into any site as long as the functions of the probe/primer are not impaired. For example, when the probe is a nucleic acid, the unsaturated functional group may be introduced into any position of the nucleic acid, but is preferably introduced into the terminus of the chain of the nucleic acid. The modified probe can be produced according to a publicly-known method. For example, the modified probe can be produced as described in International Publication WO02/062817 pamphlet.

### <Gel held by DNA microarray>

At the time of using a DNA microarray, the entire reaction system is subjected to a heat cycle for performing a nucleic acid amplification reaction (PCR or the like). In this regard, any gel, which is not chemically or physically changed by heat to cause elution of a probe into a liquid phase reaction solution, and which is not melted by heat, and whose form is not changed by heat to the extent that detection cannot be performed, can be used. Specifically, at the time of performing a PCR reaction, the temperature of the entire reaction system reaches about 94°C, and there is no problem if melting of the gel itself and elution of the probe immobilized to the gel do not occur at this time.

As a monomer to be used for preparation of a gel, for example, a "substituted (meth)acrylamide derivative" can be used. The derivative refers to a compound represented by general formula (I) below:

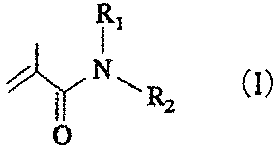

In the general formula (I), R₁ and R₂ each independently represent a hydrogen atom or a saturated alkyl group.

The substituted (meth)acrylamide derivative represented by the general formula (I) is not limited, and examples thereof include metacrylamide, N-methlylacrylamide, N,N-dimethylacrylamide, N-Ethylacrylamide, N-Cyclopropylacrylamide, N-isopropylacrylamide, N,N-Diethylacrylamide, N-Methyl-N-Ethylacrylamide, N-Methyl-N-Isopropylacrylamide and N-Methyl-N-n-propylacrylamide.

The composition of the gel is not particularly limited. For example, in addition to the above-described substituted (meth)acrylamide derivative, a monomer such as N-acryloyl aminoethoxyethanol, N-acryloyl aminopropanol, N-methylolacrylamide, N-vinyl pyrrolidone, hydroxyethyl methacrylate, (meth)acrylic acid and allyldextrin can be used. Moreover, a gel obtained by copolymerization of one or more types of substituted (meth)acrylamide derivatives and a multifunctional monomer such as methylenebis(meth)acrylamide and polyethylene glycol di(meth)acrylate can also be used. In addition, for example, a gel containing agarose, alginic acid, dextran, polyvinyl alcohol, polyethylene glycol, derivatives thereof and the like, or a gel in which these substances are crosslinked by a cross-linking agent can be used.

The cross-linking agent is a multifunctional monomer having two or more ethylenically unsaturated bonds. Examples thereof include N,N'-methylenebis acrylamide, N,N'-diallyl(1,2-hydroxyethylene)-bis-acrylamide, N,N'-cystamine-bis-acrylamide, N-acryloyltris(hydroxymethyl)aminomethane and polyethylene glycol dimethacrylate. Preferably, N,N'-methylenebis acrylamide is used.

Hereinafter, the copolymerization reaction will be described.

The modified probe to be used at the time of the copolymerization reaction is preferably used with a number of moles which is 1/10 or less of the total number of moles of the monomer to be used for preparation of the gel, but is not particularly limited as long as it is within the range in which the conditions of resistance characteristics at the time of the aforementioned heat cycle can be met.

The molar ratio between the amount of the cross-linking agent used in the copolymerization reaction and the total amount of the monomer used for preparation of the gel (substituted (meth)acrylamide derivative, etc.) (monomer: cross-linking agent) is preferably in the range of 8:2 to 500:1. When the molar ratio is larger than 8:2, the gel network becomes unhomogeneous and a white turbidity is easily yielded. When the molar ratio is less than 500:1, there is a possibility that the structure as the gel is no loner maintained.

The polymerization initiator is not limited as long as decomposition of the probe does not significantly occur at the time of the polymerization reaction, but preferred polymerization initiators are 2,2'-azobis[2-(2-imidazoline-2-yl)propane]dihydrochloride, APS (ammonium persulfate), KPS (potassium persulfate) and the like. Further, when using APS or KPS as the polymerization initiator, TEMED (tetramethylenediamine) can be used as a polymerization accelerator.

The reaction temperature at the time of the polymerization reaction is not limited, but is preferably 40°C or higher when an azo-based polymerization initiator is used. Further, when using APS or KPS solely, the reaction temperature is preferably 50°C or higher. When using TEMED together with APS or KPS, the reaction temperature is preferably in the range of room temperature to 30°C.

### <Setting of gel concentration>

The polymer concentration (gel concentration) in the gel formed by the above-described copolymerization reaction can be suitably adjusted and set depending on the composition of the gel and the size (base length) of a nucleic acid as a detection target. In the present invention, it is possible to provide a plurality of sections (spots) of gel on the same array, and in this case, it is possible to provide a plurality of types of spot gels with different gel concentrations on the same array.

The specific gel concentration is not particularly limited, but is preferably more than 2 % by mass and less than 5 % by mass, more preferably 2.5 to 4.5 % by mass, even more preferably 2.5 to 4 % by mass, and particularly preferably 2.8 to 3.8 % by mass. When the gel concentration is within the above-described range, it is preferred when specifically detecting a nucleic acid having a base length of 50 bases or more and less than 3000 bases. The base length is more preferably 50 to 2000 bases, even more preferably 100 to 2000 bases, still more preferably 100 to 1500 bases, particularly preferably 100 to 1200 bases, and most preferably 100 to 1000 bases. Further, the above-described range of the gel concentration is particularly preferred when, for example, N,N-dimethylacrylamide is included in the monomer constituting the gel.

### <Preparation of substrate on which probe-immobilized gel is mounted>

The probe-immobilized gel to be used in the method for detecting a nucleic acid of the present invention may be in any form without limitation as long as it can be brought into contact with a reaction solution containing a template nucleic acid, etc. to perform a nucleic acid amplification reaction (PCR, etc.). However, basically, a form in which the probe-immobilized gel is mounted on a substrate of some kind is preferred.

For example, it is possible to produce a gel-held tubular body by filling the hollow portion of a tubular body with a gel. The tubular body can be used as a tool for detection of genetic mutation and the like as described, for example, in Japanese Laid-Open Patent Publication No. H03-47097. The gel can be held in the hollow portion of the tubular body in a manner similar to that for the preparation of a capillary column used for capillary gel electrophoresis.

Further, it is also possible to produce a microarray on which a plurality of types of gels (probe-immobilized gels) with different gel concentrations are carried. For example, a DNA microarray can be produced by spotting monomer solutions containing a probe (a plurality of types of gel precursor solutions with different monomer concentrations) prior to polymerization or immediately after initiation of polymerization in predetermined sections of a planar substrate (see Japanese National-phase PCT Laid-Open Patent Publication No. H06-507486 and US Patent No. 5,770,721). When each section of the above-described substrate is formed by a groove (including a well) or through-hole, the monomer solutions containing a probe prior to polymerization or immediately after initiation of polymerization are added to such grooves or through-holes and a polymerization reaction is performed in the sections, thereby preparing a DNA microarray in which the probe-immobilized gel is held (mounted) in the grooves or through-holes of the substrate (see Japanese Laid-Open Patent Publication No. 2000-60554).

Moreover, a DNA microarray can be prepared by bundling a plurality of tubular bodies holding a probe-immobilized gel and repeatedly slicing the bundled product in the direction crossing the longitudinal direction of the tubular bodies into thin sections (see International Publication WO00/53736 pamphlet). It is also possible to bundle a plurality of tubular bodies in advance and then allow each hollow portion of the bundled product to hold a gel, followed by the above-described slicing into thin sections. In this case, a DNA microarray can be produced by performing the below-described steps (1) to (4) in sequence.

(1) A step wherein a plurality of tubular bodies are bundled such that the longitudinal directions thereof correspond to each other.
(2) A step wherein the hollow portion of each of the tubular bodies of the bundled product is filled with a solution containing a monomer to be used for gel preparation, a cross-linking agent and a probe.
(3) A step wherein a copolymerization reaction is performed in the hollow portion.
(4) A step wherein the bundled product is sliced in the direction crossing the longitudinal direction of the bundled product.

Examples of the tubular body include a glass tube, a stainless tube and a hollow fiber. In consideration of processability and ease of handling, it is particularly preferred to use a hollow fiber. In this regard, as a method for producing a microarray using a hollow fiber as the tubular body, the method in which the below-described steps (a) to (d) are performed in sequence can be specifically exemplified.
(a) A step wherein a plurality of hollow fibers are three-dimensionally arranged so that the fiber axial directions of the hollow fibers become the same, and wherein the arrangement is fixed with a resin to produce a hollow fiber bundle.
(b) A step wherein a plurality of types of gel precursor solutions with different monomer concentrations containing a probe are introduced into the respective hollow portions of the hollow fibers of the hollow fiber bundle.
(c) A step wherein the gel precursor solutions introduced into the hollow portions are reacted (copolymerization reaction) to hold a gel-like product comprising the probe (probe-immobilized gel) in the hollow portions of the hollow fibers.
(d) A step wherein the hollow fiber bundle is sliced in the direction crossing the longitudinal direction of the fibers into thin sections.

In this way, a DNA microarray, in which a gel to which a probe (probe nucleic acid or the like) is immobilized is held in the hollow portion of each of many hollow fibers arranged, can be prepared.

### <Method of using DNA microarray>

Hereinafter, the method of using the aforementioned DNA microarray will be described.

After the preparation of the DNA microarray, a nucleic acid can be detected according to the following procedures (a) to (d):
(a) a step wherein the aforementioned probe-immobilized gel is brought into contact with a reaction solution which contains a specimen (a nucleic acid that serves as a template for nucleic acid amplification), a primer set for nucleic acid amplification, a nucleotide unit and a DNA extension enzyme. In the step, the probe-immobilized gel may be in the state of being held by a substrate such as a DNA microarray, or may be in the state of being not held by the substrate or the like;
(b) a step wherein the gel and the reaction solution are subjected to a predetermined heat cycle for performing a nucleic acid amplification reaction (PCR, etc.). In the step, when the gel is a gel in the state of being held by a substrate such as a DNA microarray, a target to be subjected to the heat cycle is usually the entire DNA microarray or the like brought into contact with the reaction solution (then the entire reaction system);
(c) a step wherein nucleic acid fragments having a specific base length are selected from among the amplified nucleic acid fragments. The selection of nucleic acid fragments having a specific base length in the step means selection of nucleic acid fragments utilizing the molecular sieving effect of the probe-immobilized gel depending on the gel concentration. Therefore, the selection is a process carried out by means of characteristics of the probe-immobilized gel after the nucleic acid amplification reaction;
(d) a step wherein the selected nucleic acid fragments are detected.

Hereinafter, the method of the above-described nucleic acid detection will be described in detail.

### <Nucleic acid (specimen) that serves as a template for nucleic acid amplification>

Specifically, the nucleic acid that serves as a template for nucleic acid amplification contained in the reaction solution is used as a specimen.

Firstly, a solution containing a nucleic acid is prepared. The solution may be a purified one or an unpurified one as long as a reaction is not inhibited at the time of the heat cycle. When purifying the solution, a biological sample or the like is collected and a nucleic acid is extracted therefrom. As a method for extracting a nucleic acid from a biological component, for example, phenol extraction, ethanol precipitation, or any extraction method can be used. When extracting mRNA, an oligo-dT column may be used. If necessary, the material is further separated and purified into DNA or RNA. More specifically, for example, a genomic DNA, or a transcription product (mRNA) or a cDNA obtained by reverse transcription thereof of a specific living organism, a genomic DNA mixture or a mixture of transcription products (mRNAs) of one or more types of living organisms or the like is included. Moreover, products obtained by subjecting these substances to an enzyme treatment or the action of a chemical substance are also included.

By means of the nucleic acid amplification reaction using these nucleic acids as templates, it is possible to amplify a nucleic acid fragment of a certain region, or to amplify a nucleic acid fragment which is contained only in a specific living organism, or to quantitate the expression level of a specific gene (mRNA). As a method for amplification of a nucleic acid, various methods such as the PCR method, the LAMP method, the ICAN method, the TRC method, the NASBA method and the PALSAR method can be used. Among them, the PCR method is preferred, but as long as there is no particular problem in the detection of a nucleic acid, any method can be used for the nucleic acid amplification reaction.

### <Primer set for nucleic acid amplification>

Usually, a nucleic acid sequence (nucleic acid fragment) as a detection target is determined in advance. That is, the chain length and the region of a nucleic acid as a detection target are determined at this point, and the composition and the concentration of a gel through which the nucleic acid having the chain length can be passed are also determined in advance. The primer set for nucleic acid amplification can be mixed with a liquid phase reaction solution in advance or can be immobilized on a spot of a DNA microarray together with a probe in advance. The primer set is determined such that the nucleotide sequence of an oligonucleotide that serves as a probe (also functions as a primer) is included in the middle of a sequence to be amplified (a region sandwiched by the primer set). The length of the primer is usually set to about 20 to 50 nucleotides, and two types of primers, i.e., a forward primer and a reverse primer (one set), or more (more than one set) of primers are usually required for one gene region to be amplified.

In order to carry out the present invention, it is required that the one or more sets of primers are mixed, and that one or more types of probes having a specific nucleotide sequence which hybridizes to the amplified product are immobilized.

In this regard, when selecting a sequence which can amplify a plurality of gene regions for one set of primers, the reaction system is more simplified. Specifically, examples thereof include the case where the sequences of 16S rRNA of a plurality of living organisms are compared to each other, primers (one set) are designed in the common region, and for every species, a probe for detecting an individual species is designed in the portion sandwiched by the primers.

Moreover, in order to facilitate the detection later, the termini of the primer set to be used can be labeled with a fluorescent substance (cy3, cy5 or the like), biotin or the like in advance. The labeling method is not particularly limited, and any method can be used as long as phenomena such as that in which an amplification reaction is significantly inhibited do not occur.

### <Nucleotide unit>

Examples of the nucleotide unit include deoxynucleotide triphosphate which is used in ordinary amplification reaction. As in the case of the primer set, it is possible to use a derivative which facilitates the detection later as the nucleotide unit, but it is preferred to use a substance which does not inhibit an amplification reaction.

### <DNA extension enzyme and others>

As the DNA extension enzyme, like those used for the ordinary PCR method, TaqDNA polymerase, TthDNA polymerase, PfuDNA polymerase and the like, which are DNA polymerases derived from heat-resistant bacteria, can be used.

Examples of specific enzymes and kits that can be used include Hot StarTaq DNA Polymerase (QIAGEN), PrimeStarMax DNA Polymerase (Takara Bio Inc.), SpeedSTAR HS DNA Polymerase (Takara Bio Inc.), KOD-Plus-Neo (Toyobo Co., Ltd.), and KAPA2G FastHotStartPCR kit (NIPPON Genetics Co., Ltd.).

### <Heat cycle>

A reaction solution containing a specimen (specimen nucleic acid) is brought into contact with a probe-immobilized gel held by a DNA microarray or the like, and then a predetermined heat cycle for performing a nucleic acid amplification reaction such as PCR is applied to the entire reaction system including the DNA microarray, etc. In this regard, bringing the probe-immobilized gel into contact with the reaction solution may be immersing the probe-immobilized gel (i.e., the DNA microarray or the like by which the gel is held) in the reaction solution or may be feeding the reaction solution to the probe-immobilized gel (or the DNA microarray or the like by which the gel is held), and is not limited.

In this regard, in the present description, it is preferred that the contact surface area of the gel on which the probe is immobilized in each section and the reaction solution (the surface area of the gel in contact with the reaction solution) (S (µm²)) is not too large relative to the volume of the gel (V (µm³)). When it is too large, there is a possibility that the molecular sieving effect of the gel (the effect that the gel itself selects the size of a nucleic acid molecule) is not sufficiently exerted. For example, in the techniques described in Non-Patent Document 6 (Gennady Yershov et al., Proc. Natl. Acad. Sci. USA, Vol. 93, pp. 4913-4918, May 1996) and Non-Patent Document 7 (A. Yu. Rubina et al., Analytical Biochemistry, Vol. 325, pp. 92-106, 2004) described above, the form of the gel is more flattened to increase the diffusional efficiency (the contact area relative to the reaction solution is increased as much as possible to size up the net-like structure of the gel). However, though the time required for hybridization can be reduced thereby, nucleic acid molecules that hybridize reach saturation in the gel in a short time, and therefore, almost no molecular sieving effect of the gel is exerted. Whereas in the present description, the ratio of the volume of the gel (V (µm³)) to the contact surface area of the gel and the reaction solution (S (µm²)) (i.e., a value obtained by dividing a value of V by a value of S (V/S)) is not limited, but is, for example, 50 or more, preferably 75 or more, more preferably 100 or more, and even more preferably 125 or more. By designing the form of the probe-immobilized gel, in particular the form of being held by a substrate (the form of being mounted) in a manner such that the value of V/S is within the above-described range, the molecular sieving effect of the gel can be sufficiently exerted, and as a result, the specificity of the detection of a nucleic acid having a desired base length can be further improved.

Note that as a container for putting both the DNA microarray or the like and the reaction solution in, for example, a product in the form of the 96-well plate having a size that conforms to the SBS standards is preferably used in consideration of the fact that many samples can be treated at a time and that wells having a size sufficient for putting the DNA microarray or the like are provided. Each well of the 96-well plate may have a square shape or round shape.

Further, regarding the bottom face of the well plate, in order to maintain thermal conductivity, it is preferred that the difference in height of the lower surface in contact with a heatsink is almost 0, and that it is thin. Further, regarding heat resistance, there is no particular problem if deformation does not occur at 103°C or higher. The thickness of the bottom portion of the well plate satisfying the above-described conditions is not limited, but is preferably 0.05 mm to 0.5 mm, more preferably 0.1 mm to 0.4 mm, and even more preferably 0.15 mm to 0.35 mm. When the thickness exceeds the upper limit, there is a possibility that thermal conductivity is reduced, and when the thickness is less than the lower limit, there is a possibility that distortion of the bottom portion of the well plate is generated.

In consideration of the matter that the detection is performed according to an optical technique after a reaction, it is preferred that the bottom face of the well plate has a higher transparency. Meanwhile it is preferred that a lid or seal is used for the top face for the purpose of sealing. The lid or seal may be made of any material which does not adversely affect the reaction system. Further, any material, which has heat resistance, chemical resistance and the like to the extent that deformation, elution of impurities, etc. do not occur at a temperature given at the time of a reaction applied, may be used. When performing a measurement from the top face using an optical measuring device, for example, an adhesive seal which is commercially available for real-time PCR or the like can be used.

As the material of the well plate, a thermoplastic resin is preferred, and a thermoplastic resin with a smaller amount of fluorescence generated can be used. By using a resin with a smaller amount of fluorescence generated, the background at the time of detection can be reduced, and therefore detection sensitivity can be further improved. Examples of the thermoplastic resin with a smaller amount of fluorescence generated that can be used include: linear polyolefins such as polyethylene and polypropylene; cyclic polyolefins; and fluorine-containing resins. Among them, saturated cyclic polyolefin is preferably used as the material of the well plate because it is particularly excellent in heat resistance, chemical resistance, low fluorescence, transparency and moldability. As a commercially available 96-well plate, for example, a 96-IQ plate (Aurora Biotechnologies) or the like can be used.

The method for controlling the temperature of a heat cycle performed in the detection method will be described below. As the temperature control, for example, by bringing the bottom face of the well plate into contact with a hot plate (heat block) adjusted to have a given temperature, the temperature of the reaction solution can be controlled. In another embodiment, it is possible to adjust the temperature of the reaction solution much more rapidly according to a method in which each of a plurality of hot plates (heat blocks) is controlled to have a predetermined different temperature in advance and a well plate moves on the hot plates in order. Further, by bringing not only the bottom face but also the top face of the well plate into contact with a hot plate (heat block), the temperature of the reaction solution can be adjusted and controlled in a shorter time.

As a temperature control device to be used for the heat cycle in this diction method, a commercially-available thermal cycler can be used, but it is preferred to use a product to which a well plate can be directly set. For example, GeneAmp 9600 and GeneAmp 9700 (Life Technologies Corporation), T Professional series (Biometra), etc. can be used, but any type may be used as long as there is no problem of thermal conductivity or the form of a lid. When a well plate for a DNA microarray and a reaction solution to be put in has a depth (thickness) that cannot be received by a thermal cycler, it is possible to scrape the top face portion away to decrease the depth and thickness for use.

### <Method for detecting amplified product>

As a method for detecting an amplified product (nucleic acid fragment amplified and selected by the molecular sieving effect of the gel), for example, a method according to color development measurement or fluorescence intensity measurement using a fluorescent substance or luminescent substance as a labeling substrate, or a method according to visual observation can be used. Specifically, the presence/absence and the quantity of the amplified product can be determined using a fluoroimaging analyzer, a CCD camera or the like. Further, desirably, by monitoring the amount of fluorescence at each spot in a time-dependent manner using an apparatus for quantitation of PCR reaction (Real Time PCR apparatus), which has been being frequently used recently, or the like, quantitation of a nucleic acid having higher reliability can be realized. Moreover, it is also possible to perform a color development on the gel using a coloring reagent or the like which may or may not utilize an enzyme reaction. In such a case, it is possible to determine the position of a detection spot on the DNA microarray macroscopically and to perform scanning using a photoscanner.

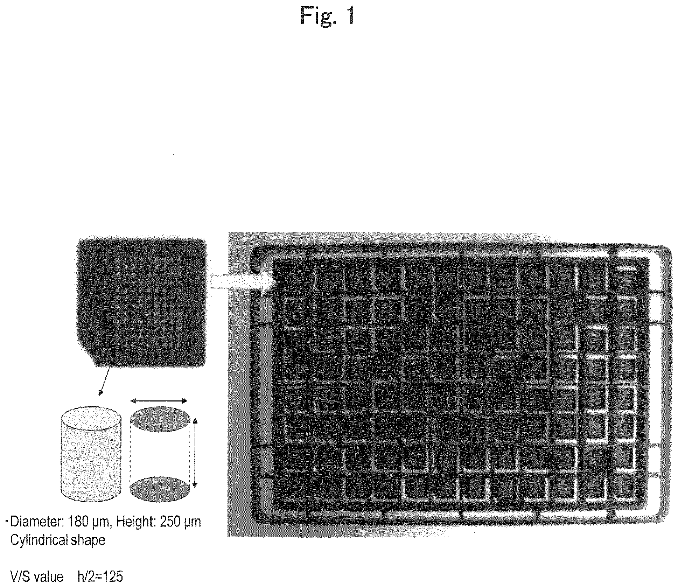

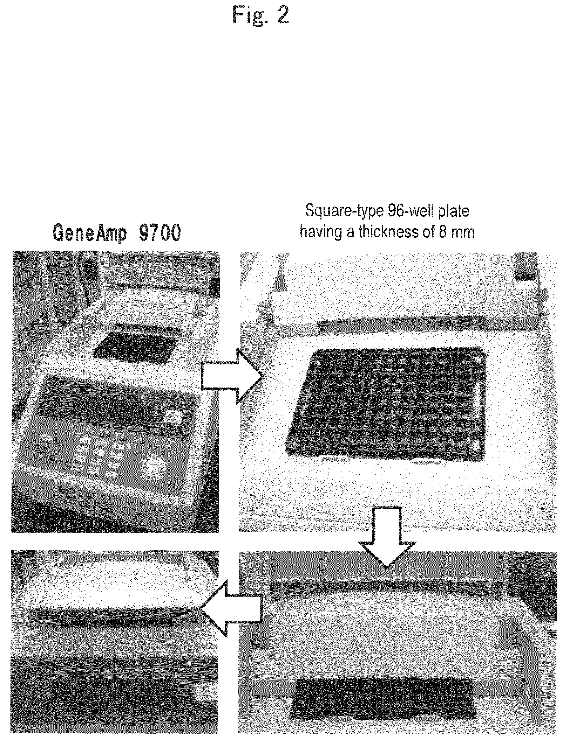

One embodiment of the method of using a DNA microarray in the present invention is shown in Figures 1 and 2. In the left upper portion of Figure 1, a 7 x 7 mm square DNA microarray is shown, and in the right portion of Figure 1, a square-type 96-well plate with 96 DNA microarrays (7 x 7 mm) being held therein is shown. In Figure 2, this is received by a thermal cycler. After a heat cycle is performed by the thermal cycler, the well plate is taken out therefrom, and the detection can be immediately made from the top or bottom face of the well plate using a CCD camera.

Next, the principle of the method for detecting a nucleic acid of the present invention will be described. A gel-utilized DNA microarray is put in a well of a well plate, and then a reaction solution is added to the well to bring the DNA microarray into contact with the reaction solution or immerse the DNA microarray in the reaction solution, followed by subjecting it to a heat cycle. In this way, a nucleic acid amplification reaction such as PCR can be carried out.

The reaction is performed by repeatedly reacting the DNA microarray and the entire reaction solution by means of a heat cycle having 3 steps (denaturation → annealing → replication) or 2 steps (denaturation → annealing/replication) at respective temperatures optimized in advance.

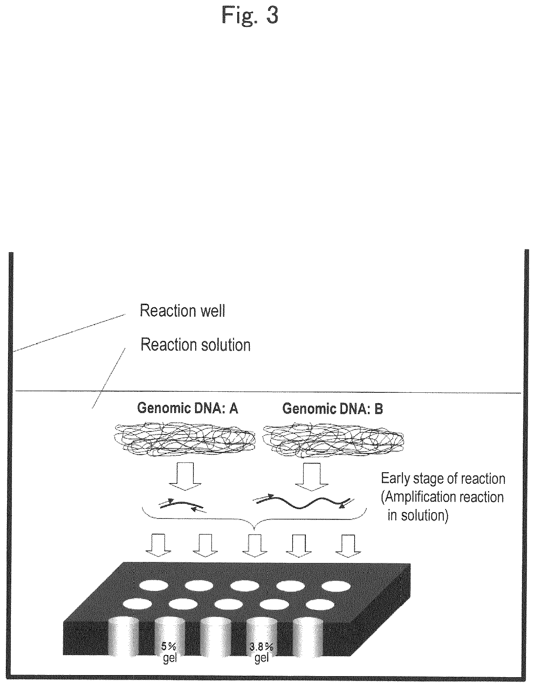

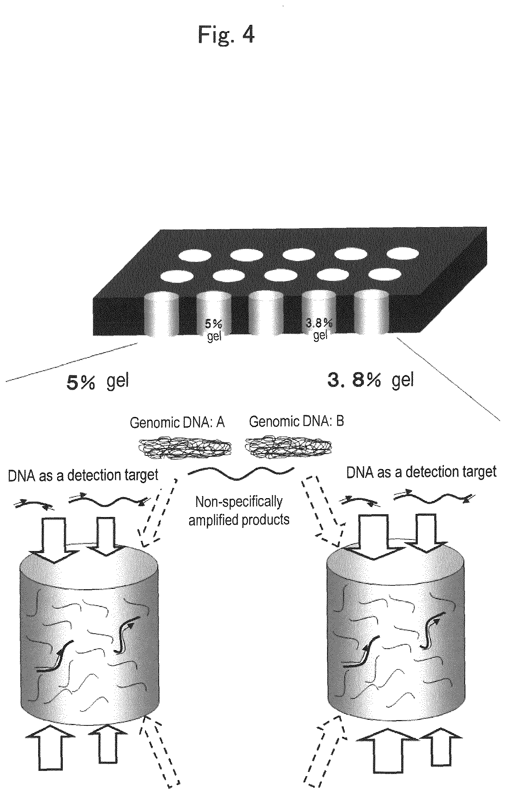

As shown in Figure 3, in the early stage of the heat cycle, a nucleic acid amplification reaction (PCR) occurs mainly in a liquid phase (in the reaction solution). At the same time, a part of amplified products (targeted nucleic acid fragments) hybridize to a probe DNA immobilized on a spot gel. At this time, large-size nucleic acids such as genomic DNA and non-specifically-amplified nucleic acids having a large size which cannot be diffused in the gel are eliminated and cannot go to the next step (Figure 4). Further, as already described, the gel concentration of the spot is set corresponding to the size (base length) of a nucleic acid as a detection target, i.e., in consideration of the size of a nucleic acid to be diffused in the gel.

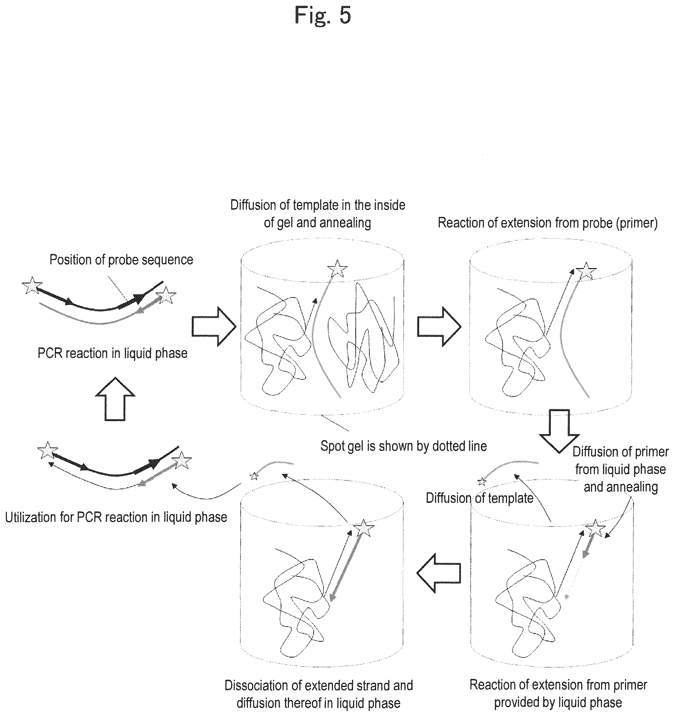

Subsequently, an extension reaction of the probe DNA is caused by a DNA polymerase contained in the reaction solution using a target DNA as a template. By the extension reaction, a region to which the primer DNA in the liquid phase can hybridize is made (the right upper portion and the right lower portion of Figure 5).

After that, by heating, dissociation of a double-stranded DNA is caused, and simultaneously, the primer from the liquid phase is annealed to the terminus of the extended probe (primer) and the amplification reaction of the opposite strand proceeds. Regarding the opposite strand, since the template ends at the 3'-terminal portion of the probe sequence, synthesis thereof is terminated without giving the full length, but by heating after that, a part of it is diffused in the liquid phase and can play a role as a template, a primer for nucleic acid amplification reaction (PCR) (the lower portion of Figure 5).

By carrying out such steps repeatedly, DNA amplification and extension of the probe DNA immobilized on the gel, hybridization of amplified products (amplified nucleic acid fragments) and the nucleic acid amplification reaction (PCR) in the liquid phase proceed simultaneously.

When the terminus of the primer to be put in the liquid phase is labeled with a fluorescent substance or the like in advance, a target DNA can be detected and quantitated by detecting the presence/absence of the label or quantitating the label at the time of the detection.

Particularly in the present invention, as shown in Figure 4, even if an unexpected amplified product is generated at the time of the nucleic acid amplification reaction (PCR), a nucleic acid having a size larger than the intended size is not easily diffused in the gel or allowed to be subjected to hybridization because of the molecular sieving effect of the gel, and therefore, reduction of the background and improvement of specificity are promoted thereby. The DNA microarray of the present disclosure is characterized in that a clear detection result is easily obtained when determining the presence/absence of a nucleic acid.

As one exemplary embodiment, the PCR method is particularly described above, but any method other than the PCR method can also be employed as long as it is a nucleic acid amplification method that is more appropriate for the reaction system for SNP detection.

The method for detecting a nucleic acid of the present invention can be applied to, for example, a basic tool for gene analysis such as analysis of single nucleotide polymorphism (SNP), microsatellite analysis, chromosome aberration analysis (CGH: Comparative Genomic Hybridization) and search of (nc) RNA with unknown functions; a custom chip for respective intended uses utilizing the tool such as a chip for gene expression analysis for each organ/disease, a mutagenicity test kit (environmental hormone), a test kit for genetically-modified foods, a kit for analysis of mitochondrial gene sequences, a kit for analysis regarding determination of parentage and criminal investigation, a kit for analysis of congenital diseases, a kit for analysis of chromosome/gene abnormality, a kit for genetic diagnosis (before implantation/before birth), a kit for analysis of gene polymorphism associated with drug reaction, a kit for analysis of gene polymorphism associated with lipid metabolism and a kit for analysis of gene polymorphism in the otolaryngological/ophthalmologic field; a diagnostic/clinical custom chip such as a chip for prediction of cancer prognosis, a chip for drug development (clinical practice/drug discovery) and a chip for health food development; and a kit for microorganism identification test such as a test in the drug/food production process such as a microbial limit test and a test of microorganisms in food/drinking water, a clinical test in the dental field such as detection of bacteria associated with decay/periodontal disease and detection of opportunistic bacteria, an environmental test in food factories/kitchens, an environmental test regarding a water quality test of beverages, public baths, well water, etc., health and hygiene such as prevention of infectious diseases/food poisoning and hygiene management for company employees, identification of common bacteria including resistant bacteria, and a clinical test of hepatitis virus, Helicobacter pylori, chlamydia associated with hepatitis, AIDS virus, SARS virus, West Nile virus, norovirus (food poisoning derived from raw oysters), influenza virus, fungus, mold, etc.

Hereinafter, the present invention will be more specifically described by way of examples. However, the present invention is not limited to these examples.

### EXAMPLES

### Example 1

### 1. Preparation of gel-held DNA microarray (preparation of DNA microarray having a plurality of spots with different gel concentrations)

A DNA microarray was produced as described below.

### 1-1. Preparation of probe

Firstly, the oligonucleotide described in SEQ ID NO: 1 was prepared for use as a probe. At this time, an oligonucleotide in which an aminohexyl group was introduced into the 5' end of an oligonucleotide was prepared. Next, the oligonucleotide was reacted with methacrylic anhydride, and then purified with HPLC and collected, thereby obtaining a 5' end vinylated oligonucleotide having the nucleotide sequence represented by SEQ ID NO: 1 below (probe (also functions as a primer)).

5'-AGGAKGTTGGCTTAGAAGCAGCCA-3' (SEQ ID NO: 1)

(The abbreviation "K" represents guanine (G) or thymine base (T).)

This oligonucleotide can hybridize to a portion of the 23S ribosomal DNA sequence (genomic DNA sequence encoding 23S ribosomal RNA) of Bacillus cereus.

### 1-2. Production of thin sheet made of a bundle of hollow fibers (DNA microarray)

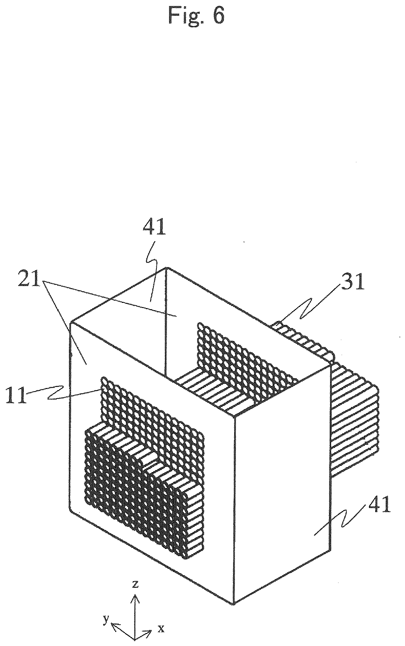

A bundle of hollow fibers was produced using a sequence immobilization device shown in Figure 6. In Figure 6, letters x, y and z represent three-dimensional axes perpendicular to one another, and the x axis matches the longitudinal direction of the fibers.

Referring to Figure 6, two porous plates (21), each of which has a thickness of 0.1 mm and has 108 pores (11) having a diameter of 0.32 mm formed therein, were prepared. The pores were arranged in 12 rows by 9 columns, and the distance between the centers of each two adjacent pores was 0.42 mm. These porous plates were stacked, and one polycarbonate hollow fiber (31) (produced by Mitsubishi Engineering-Plastics Corporation; having 1% by mass of carbon black added thereto) was passed through each of all the pores.

The two porous plates were moved away from each other in the state where a tensile force of 0.1 N was applied to each fiber in the x axis direction, and the two porous plates were respectively fixed at two positions, i.e., a position of 20 mm away from one end of the hollow fibers and a position of 100 mm away from the one end of the hollow fibers. That is, the two porous plates were separated from each other by 80 mm.

Next, the space between the two porous plates was enclosed by a plate-like body (41) on three sides thereof. Thus, a vessel which is open only at the top was obtained.

Then, a resin material was injected into the vessel from the top opening. As the resin material, a polyurethane resin adhesive (Nipporan 4276, Coronate 4403, manufactured by Nippon Polyurethane Industry Co., Ltd.) having 2.5% by mass of carbon black added thereto with respect to the total amount of the adhesive was used. The resin material was kept still at 25°C for 1 week to be cured. Then, the porous plates and the plate-like body were removed to obtain a hollow fiber bundle.

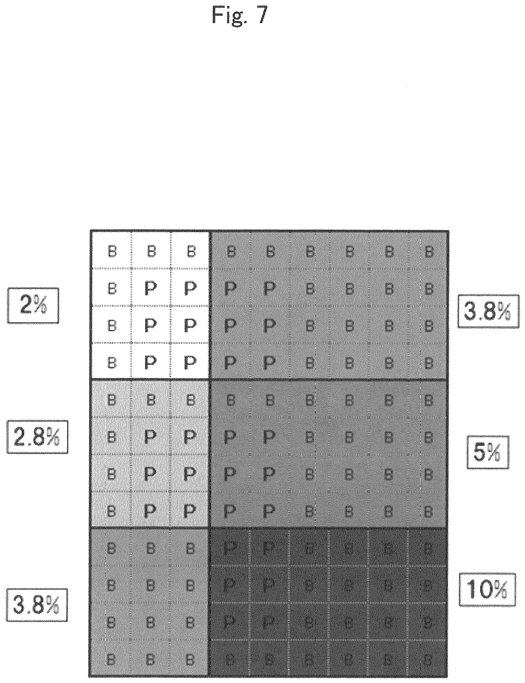

As gel precursor polymerizable solutions, solutions were prepared by mixing at the mass ratios and the concentrations shown in Table 1 below. As a nucleic acid probe, the aforementioned oligonucleotide represented by SEQ ID NO: 1 was used, and 5 types of gel precursor polymerizable solutions were prepared. At portions on which no probe was mounted, water was used instead of the nucleic acid probe. Respective spots and gel concentrations thereof, and arrangement of probes are shown in Figure 7. In Figure 7, sections with different colors (from faint color to deep color) show the difference of gel concentration. At sections represented by "P", the nucleic acid probe is immobilized, and at sections represented by "B", only the gel is present and no nucleic acid probe is contained.

**Table 1]**

| | | | | | |
|---|---|---|---|---|---|
| Gel concentration (% by mass) | 10.00% | 5.00% | 3.80% | 2.80% | 2.00% |
| N,N-dimethylacrylamide (monomer) (% by mass) | 9.00% | 4.50% | 3.42% | 2.52% | 1.80% |
| N,N -methylenebisacrylamide (cross-linking agent) (% by mass) | 1.00% | 0.50% | 0.38% | 0.28% | 0.20% |
| 2,2'-azobis[2-(2-imidazoline-2-yl)propane] dihydrochloride (VA-044, initiator) (% by mass) | 0.01% | 0.01% | 0.01% | 0.01% | 0.01% |
| Milli-Q water (% by mass) | 89.99% | 94.99% | 96.19% | 97.19% | 97.99% |
| Monomer/cross-linking agent (molar ratio) | 14/1 | 14/1 | 14/1 | 14/1 | 14/1 |
| Nucleic acid probe (final concentration) | 5 pmol/µl | 5 pmol/µl | 5 pmol/µl | 5 pmol/µl | 5 pmol/µl |

Next, the gel precursor polymerizable solution containing the nucleic acid probe was set in a desiccator. After the inner pressure of the desiccator was set to a reduced level, one end of the hollow fiber bundle which is not fixed was immersed in the solution. Nitrogen gas was injected into the desiccator, and the gel precursor polymerizable solution containing the nucleic acid probe was introduced into the hollow portions of the hollow fibers. Then, the temperature inside the vessel was set to 50°C, and the polymerization was allowed to proceed for 3 hours.

Thus, a hollow fiber bundle having the nucleic acid probes retained in the hollow portions of the hollow fibers by the gel-type material was obtained.

Next, the obtained hollow fiber bundle was appropriately sliced in the direction perpendicular to the longitudinal direction of the fibers using a microtome to produce 300 thin sheets (DNA microarrays) having a thickness of 0.25 mm (see the left upper portion of Figure 1).

In this working example, in the probe-immobilized gel in each section, the ratio of the volume of the gel (V (µm³)) to the contact surface area of the gel and the reaction solution (S (µm²)) (i.e., a value obtained by dividing a value of V by a value of S (V/S)) was 125 as shown in the left lower portion of Figure 1.

### 2. Confirmation of the improvement of specificity utilizing the molecular sieving effect of gel

### 2-1. Preparation of template DNAs with different chain lengths

By utilizing the ATCC distribution service provided by Summit Pharmaceuticals International Corporation, the genomic DNA of Bacillus cereus was purchased (Accession No: ATCC 14579) to be used as a template for PCR reaction.

In order to obtain 4 types of nucleic acid fragments with different base lengths, each of which includes, in the middle thereof, the sequence of the probe mounted on the microarray (i.e., 5'-AGGAKGTTGGCTTAGAAGCAGCCA-3' (SEQ ID NO: 1)) and a nucleotide sequence derived from the 23S ribosomal DNA sequence (genomic DNA sequence encoding 23S ribosomal RNA) of Bacillus cereus, 4 pairs of primers for the above-described template were designed.

The designed 4 primer sets (forward (F) primer and reverse (R) primer) are shown below.

### Primers for obtaining PCR product of 123 bp

### - F primer (i)

PrimerFP123bp_cereus: 5'-GTATTAAGTGGAAAAGGATGTGGAGTTGC-3' (SEQ ID NO: 2)

### - R primer (i)

PrimerRP123bp_cereus: 5'-CCGGTACATTTTCGGCGCAGAGTC-3' (SEQ ID NO: 3)

### Primers for obtaining PCR product of 413 bp

### - F primer (ii)

PrimerFP413bp _cereus: 5'-AACTCCGAATGCCAATGACTTATCCTTAG-3' (SEQ ID NO: 4)

### - R primer (ii)

PrimerRP413bp_cereus: 5'-AGCCTTCCTCAGGAAACCTTAGGCA-3' (SEQ ID NO: 5)

### Primers for obtaining PCR product of 827 bp

### - F primer (iii)

PrimerFP827bp_cereus: 5'-AACTCCGAATGCCAATGACTTATCCTTAG-3' (SEQ ID NO: 6)

### - R primer (iii)

PrimerRP827bp _cereus: 5'-TTCACTGCGGCTTTCCGTTAAGAAAGCA-3' (SEQ ID NO: 7)

### Primers for obtaining PCR product of 1191 bp

### - F primer (iv)

PrimerFP1191bp_cereus: 5'-AACTCCGAATGCCAATGACTTATCCTTAG-3' (SEQ ID NO: 8)

### - R primer (iv)

PrimerRP1191bp_cereus: 5'-CCGCCTATCCTGTACAAACTGTACCAA-3' (SEQ ID NO: 9)

As the 23S ribosomal DNA sequence (genomic DNA sequence encoding 23S ribosomal RNA) of Bacillus cereus, the sequence registered as Accession No.: AJ310099.1 in the GenBank database (http://www.ncbi.nlm.nih.gov/genbank/) provided by the National Center for Biotechnology Information (NCBI) was utilized. The nucleotide sequence of the 23S ribosomal DNA sequence (genomic DNA sequence encoding 23S ribosomal RNA) of Bacillus cereus (SEQ ID NO: 10) is shown below. In the nucleotide sequence, the double-underlined portion is a nucleotide sequence which can hybridize to the probe (SEQ ID NO: 1) (since it is double-stranded, the opposite strand (complementary strand) hybridizes).

Bacillus cereus AJ310099.1 23S rRNA gene (SEQ ID NO: 10)

### <PCR reaction>

PCR reactions were performed by using the genomic DNA of Bacillus cereus (50 ng/µL) as a template and using the aforementioned 4 primer sets respectively. For the PCR reactions, an AmpDirect kit (Shimadzu Corporation) was used.

### <Composition of PCR reaction solution>

| | |
|---|---|
| Solution of genomic DNA of Bacillus cereus (50 ng/µL) | 1 µL |
| F primers (i) to (iv) (20 µM) | 0.5 µL |
| R primers (i) to (iv) (20 µM) | 0.5 µL |
| 2 × AmpDirect buffer | 50 µL |
| BioTaq (provided with AmpDirect kit) | 1 µL |
| Milli-Q water | 47 µL |
| Total | 100 µL |

GeneAmp 9600 thermal cycler was used for the PCR reactions. The temperature conditions were as described below.

### <Temperature conditions for PCR reaction>

| | |
|---|---|
| 95°C | 10 minutes |
| (94°C for 30 seconds, 64°C for 1 minute, 72°C for 30 seconds) × 35 cycles | |
| 72°C | 1 minute |
| 4°C | maintained after the completion of reaction |

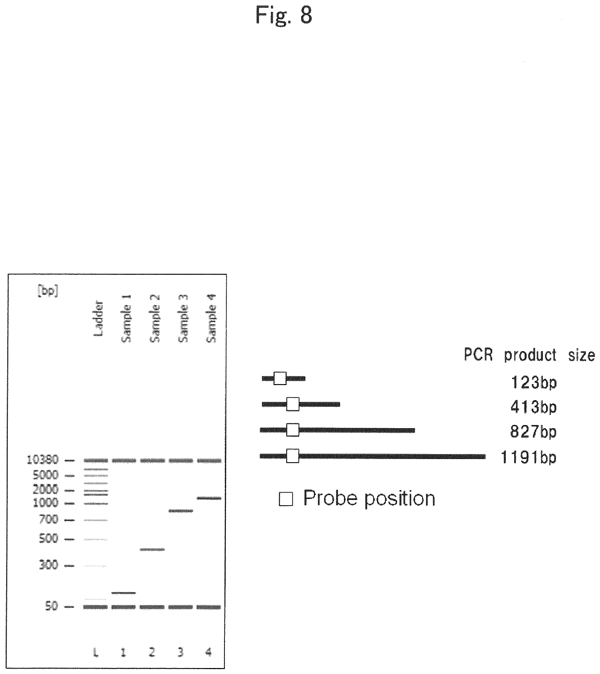

After the reaction, purification was carried out using MinElute PCR purification kit (Qiagen), and elution was carried out with 14 µL Milli-Q water. After that, electrophoresis was carried out using Bioanalyzer of Agilent, and a PCR product having a desired size was confirmed. Figure 8 shows results of electrophoresis and a schematic view of the position corresponding to the probe sequence in the middle of each of the product. In Figure 8, Lane L is a size marker, and Lanes 1-4 are PCR products of 123 bp, 413 bp, 827 bp and 1191 bp in this order.

The absorbance of each of the obtained PCR products was measured and the concentration was calculated, and the concentration of each product was adjusted to 5 fmol/µL.

### 2-2. PCR reaction in gel-held DNA microarray

A gel-held DNA microarray utilizing hollow fibers (see the left portion of Figure 1) was put into a well of a 96-IQ plate manufactured by Aurora Biotechnologies adjusted by cutting work to have a thickness of 8 mm, and 100 µL of a PCR reaction solution having the below-described composition was added thereto.

### <Composition of PCR reaction solution>

| | |
|---|---|
| Solution of PCR product of 123 bp (5 fmol/µL) | 1 µL |
| 5' end cy5-labeled F primer (i) (20 µM) | 0.5 µL |
| 5' end cy5-labeled R primer (i) (20 µM) | 0.5 µL |
| 2 × AmpDirect buffer | 50 µL |
| BioTaq (provided with AmpDirect kit) | 1 µL |
| Milli-Q water | 47 µL |
| Total | 100 µL |

After the reaction solution was added, the top was sealed with an adhesive seal for realtime PCR (ABI PRISM Optical Adhesive Covers, Applied Biosystems Inc., #4311971) to prevent leakage of the reaction solution.

Subsequently, the 96-well plate was set in the thermal cycler GeneAmp 9700 (0.2 mL block). At the time of setting, an aluminum plate having a thickness of 0.8 mm and having the same size as the bottom face of the well plate (11.5 × 7.5 cm) was placed, and the well plate was placed thereon. In addition, Microseal 'P+' Sealing Pad (Bio-Rad) was put thereon, and the lid was slid slowly so as not to cause position gap and the lever was pulled down to maintain the state of being pressed from above.

In the program of the thermal cycler, temperature setting was made as described below, and the entire reaction system was subjected to a heat cycle treatment. A heat cycle program having 40 cycles in total was conducted.

### <Heat cycle>

| | |
|---|---|
| 94°C | 7 minutes |
| (92°C for 30 seconds, 60°C for 1 minute, 72°C for 1 minute) × 20 cycles | |
| (92°C for 30 seconds, 60°C for 30 seconds, 72°C for 30 seconds) × 20 cycles | |
| 4°C | maintained after the completion of reaction |

After the completion of the PCR reaction, the well plate was taken out from the apparatus, and the total amount of the PCR reaction solution was removed by pipetting. Then purification was carried out using MinElute PCR purification kit (Qiagen), and elution was carried out with 14 µL Milli-Q water. The concentration was measured and it was 156 nmol/µL.

The DNA microarray was rinsed with 200 µL of TN buffer twice by pipetting, and then sealed with an adhesive seal and left at 50°C for 20 minutes. After that, the total amount, i.e., 200 µL of TN buffer was removed by pipetting, and the DNA microarray was rinsed with 200 µL of TN buffer twice by pipetting. After that, the total amount was removed, and 100 µL of TN buffer was added, followed by detection operation.

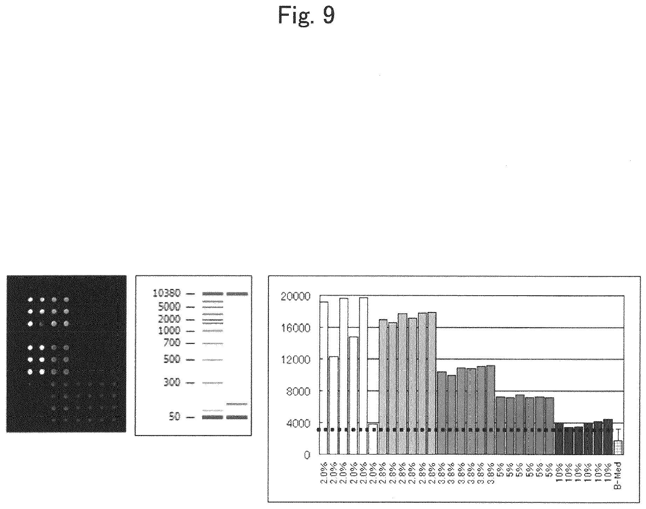

In the detection operation, a cooled CCD camera-type automatic detection apparatus for DNA microarray was used. Imaging of the DNA microarray was performed from above the well with an exposure time of 1 second, and a cy5 fluorescence signal at each spot was detected. Results of the detection are shown in Figure 9.

When the fluorescence intensity of the spot was quantitated as the median value of fluorescence intensities in the spot, gel concentration-dependent fluorescence intensity was shown. In the graph in Figure 9, B-Med at the far right is the fluorescence intensity of the blank spot. It was determined whether or not the detection was successful using the cutoff value: the fluorescence intensity of the blank spot + 2×the standard deviation of the blank spot. As a result, in the case of 123 bp, it was determined that the detection was barely successful at the spots of all the gel concentrations. However, in the case of gel of 2% by mass, significant variation of the fluorescence intensity was observed because of dropping off from a spot and a poor form of gel.

According to the results, by arranging the spot with the gel concentration being set to correspond to the size of the nucleic acid as a detection target utilizing the molecular sieving effect of the gel, the fluorescence intensity was decreased in a gel concentration-dependent manner, and selection depending on the size was observed.

It was found that by the double action, i.e., selection of the nucleic acid by the probe and selection of the size of the nucleic acid in the gel concentration-dependent manner, a DNA microarray, which enables a more specific detection compared to a flat plate-like array, can be provided.

### Example 2

The same operation as that of Example 1 was conducted except that the composition of the PCR reaction solution was changed as described below in 2-2 (PCR reaction in gel-held DNA microarray) of Example 1.

### <Composition of PCR reaction solution>

| | |
|---|---|
| Solution of PCR product of 413 bp (5 fmol/µL) | 1 µL |
| 5' end cy5-labeled F primer (ii) (20 µM) | 0.5 µL |
| 5' end cy5-labeled R primer (ii) (20 µM) | 0.5 µL |
| 2 × AmpDirect buffer | 50 µL |
| BioTaq (provided with AmpDirect kit) | 1 µL |
| Milli-Q water | 47 µL |
| Total | 100 µL |

In the same manner as Example 1, after the completion of the PCR reaction, the well plate was taken out from the apparatus, and the total amount of the PCR reaction solution was removed by pipetting. Then purification was carried out using MinElute PCR purification kit (Qiagen), and elution was carried out with 14 µL Milli-Q water. The concentration was measured and it was 401 nmol/µL.

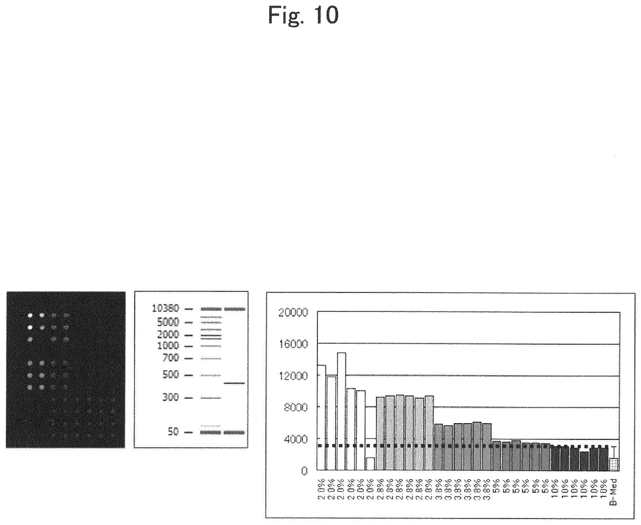

In the same manner as Example 1, a cy5 fluorescence signal at each spot was detected. Results of the detection are shown in Figure 10.

When the fluorescence intensity of the spot was quantitated as the median value of fluorescence intensities in the spot, gel concentration-dependent fluorescence intensity was shown. In the same manner as Example 1, it was determined whether or not the detection was successful using the cutoff value: the fluorescence intensity of the blank spot + 2×the standard deviation of the blank spot. As a result, in the case of 413 bp, it was determined that the detection cannot be performed at the spot with gel of 10% by mass, but it was shown that the detection can be sufficiently performed at the spot with gel of 2.8% by mass and the spot with gel of 3.8% by mass. Further, in the case of gel of 2% by mass, significant variation of the fluorescence intensity was observed because of dropping off from a spot and a poor form of gel.

According to the results, by arranging the spot with the gel concentration being set to correspond to the size of the nucleic acid as a detection target utilizing the molecular sieving effect of the gel, the fluorescence intensity was decreased in a gel concentration-dependent manner, and selection depending on the size was observed.

It was found that by the double action, i.e., selection of the nucleic acid by the probe and selection of the size of the nucleic acid in the gel concentration-dependent manner, a DNA microarray, which enables a more specific detection compared to a flat plate-like array, can be provided.

### Example 3

The same operation as that of Example 1 was conducted except that the composition of the PCR reaction solution was changed as described below in 2-2 (PCR reaction in gel-held DNA microarray) of Example 1.

### <Composition of PCR reaction solution>

| | |
|---|---|
| Solution of PCR product of 827 bp (5 fmol/µL) | 1 µL |
| Solution of PCR product of 1191 bp (5 fmol/µL) | 1 µL |
| 5' end cy5-labeled F primer (iii) (20 µM) | 0.5 µL |
| 5' end cy5-labeled R primer (iii) (20 µM) | 0.5 µL |
| 5' end cy5-labeled F primer (iv) (20 µM) | 0.5 µL |
| 5' end cy5-labeled R primer (iv) (20 µM) | 0.5 µL |
| 2 × AmpDirect buffer | 50 µL |
| BioTaq (provided with AmpDirect kit) | 1 µL |
| Milli-Q water | 47 µL |
| Total | 100 µL |

In the same manner as Example 1, after the completion of the PCR reaction, the well plate was taken out from the apparatus, and the total amount of the PCR reaction solution was removed by pipetting. Then purification was carried out using MinElute PCR purification kit (Qiagen), and elution was carried out with 14 µL Milli-Q water. The concentration was measured, and the product of 827 bp was 147.7 nmol/µL and the product of 1191 bp was 70.6 nmol/µL (converted based on the band of electrophoresis).

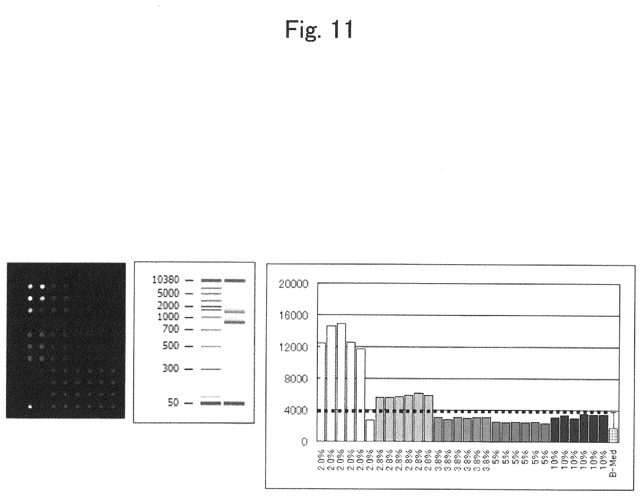

In the same manner as Example 1, a cy5 fluorescence signal at each spot was detected. Results of the detection are shown in Figure 11.

When the fluorescence intensity of the spot was quantitated as the median value of fluorescence intensities in the spot, gel concentration-dependent fluorescence intensity was shown. In the same manner as Example 1, it was determined whether or not the detection was successful using the cutoff value: the fluorescence intensity of the blank spot + 2×the standard deviation of the blank spot. As a result, in the case of the mixture of 827 bp and 1191 bp, it was determined that the detection cannot be performed at the spots with gel of from 3.8% by mass to 10% by mass, but it was shown that the detection can be sufficiently performed at the spot with gel of 2.8% by mass. In the case of gel of 2% by mass, as in the case of Examples 1 and 2, dropping off from a spot was observed. Further, variation of the fluorescence intensity was observed.

According to the results, by arranging the spot with the gel concentration being set to correspond to the size of the nucleic acid as a detection target utilizing the molecular sieving effect of the gel, the fluorescence intensity was decreased in a gel concentration-dependent manner, and selection depending on the size was observed.

It was found that by the double action, i.e., selection of the nucleic acid by the probe and selection of the size of the nucleic acid in the gel concentration-dependent manner, a DNA microarray, which enables a more specific detection compared to a flat plate-like array, can be provided.

### [Comparative Example 1]

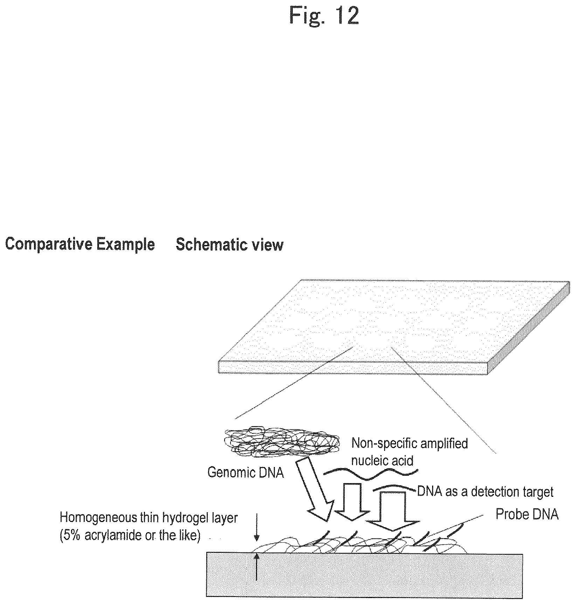

A commercially-available hydrogel-coated slide (CodeLink (registered trademark) Activated Microarray Slides (Surmodics, Inc. #DN01-0025)) was cut into a 7 x 7 mm square, and oligo DNA was spotted thereon to prepare a flat plate-like DNA array (Figure 12). The array was prepared according to the method in Example 1 of Japanese Laid-Open Patent Publication No. 2006-174788.

The prepared array was put into a well of a square-type 96-well plate as in the case of Example 1, and it was immersed in 100 µL of a PCR reaction solution to perform a PCR reaction.

In the same manner as Examples 1 and 2, the PCR reaction experiment was conducted for every different size (123 bp, 413 bp, 827 bp and 1191 bp), and a cy5 fluorescence signal at each spot was detected. Results of the detection (median value of fluorescence intensities in the spot) are shown in Table 2 below.

**[Table 2]**

| Size of PCR product used as template | Median value of fluorescence intensities |
|---|---|
| 123 bp | 12304 |
| 413 bp | 11245 |
| 827 bp | 11025 |
| 1191 bp | 12031 |

Since the array in which the probe (also functions as the primer) is immobilized on the surface of the substrate does not have the molecular sieving effect, the same level of signal intensity was detected by using any size of template. That is, it was thought that if a genomic DNA or a non-specific amplified product having a large size is produced/mixed, the condition is easily affected by non-specific hybridization thereof.

### INDUSTRIAL APPLICABILITY

The method for detecting a nucleic acid, the DNA microarray to be used in the detection method and the like of the present invention are suitable, for example, for the treatment of a large amount of specimen, and have excellent practicability and utility.

### EXPLANATIONS OF LETTERS OR NUMERALS

- 11: pore
- 21: porous plate
- 31: hollow fiber
- 41: plate-like body

### ACCESSION NUMBER

Accession No: ATCC 14579

### SEQUENCE LISTING FREE TEXT

SEQ ID NO: 1: synthetic DNA
SEQ ID NO: 2: synthetic DNA
SEQ ID NO: 3: synthetic DNA
SEQ ID NO: 5: synthetic DNA
SEQ ID NO: 6: synthetic DNA
SEQ ID NO: 8: synthetic DNA
SEQ ID NO: 9: synthetic DNA

## Claims

1. A method for detecting a nucleic acid, which comprises:
(a) a step wherein a gel, on which a nucleic acid probe is immobilized, is brought into contact with a reaction solution which contains a nucleic acid that serves as a template for nucleic acid amplification, a primer set for nucleic acid amplification, a nucleotide unit and a DNA extension enzyme;
(b) a step wherein the gel and the reaction solution are subjected to a heat cycle for performing a nucleic acid amplification reaction;
(c) a step wherein nucleic acid fragments having a specific base length are selected from among the amplified nucleic acid fragments, wherein the selecting of the nucleic acid fragments having the specific base length occurs by utilizing a molecular sieving effect of the probe-immobilized gels; and
(d) a step wherein the selected nucleic acid fragments are detected;
wherein the ratio (V/S) of the volume of the gel (V (µm³)) to the contact surface area of the gel on which the probe is immobilized and the reaction solution (S (µm²)) is 50 or more;
wherein the concentration of the gel on which the probe is immobilized is more than 2% by mass and less than 5% by mass;
wherein the gel on which the probe is immobilized comprises a substituted (meth)acrylamide derivative and/or an agarose derivative; and
wherein the primer set is determined such that the nucleotide sequence of an oligonucleotide that serves as a probe is included in the middle of a sequence to be amplified.

2. The method according to claim 1, wherein the gel on which the probe is immobilized is held in a well or through-hole in a substrate.

## Patentansprüche

1. Verfahren zum Detektieren einer Nukleinsäure, welches umfasst:
(a) einen Schritt, worin ein Gel, auf dem eine Nukleinsäuresonde (nucleic acid probe) immobilisiert ist, mit einer Reaktionslösung in Kontakt gebracht wird, die eine Nukleinsäure, die als eine Matrize (template) zur Vervielfältigung von Nukleinsäure dient, ein Primerset zur Vervielfältigung von Nukleinsäure, eine Nukleotideinheit und ein DNA-Verlängerungsenzym enthält;
(b) einen Schritt, worin das Gel und die Reaktionslösung einem Hitzezyklus ausgesetzt werden, um eine Nukleinsäurevervielfältigungsreaktion durchzuführen;
(c) einen Schritt, worin Nukleinsäurefragmente mit einer spezifischen Basenlänge ausgewählt werden aus den vervielfältigten Nukleinsäurefragmenten, wobei das Auswählen der Nukleinsäurefragmente mit der spezifischen Basenlänge durch Verwendung einer Molekularsiebwirkung der Sonden-immobilisierten Gele geschieht; und
(d) einen Schritt, worin die ausgewählten Nukleinsäurefragmente detektiert werden;
wobei das Verhältnis (V/S) des Gelvolumens (V(µm³)) zum Kontaktoberflächenbereich des Gels, auf dem die Sonde immobilisiert ist und der Reaktionslösung (S(µm²)) 50 oder mehr ist;
wobei die Konzentration des Gels, auf dem die Sonde immobilisiert ist, mehr als 2 Massen-% und weniger als 5 Massen-% ist;
wobei das Gel, auf dem die Sonde immobilisiert ist, ein substituiertes (Meth-)Acrylamid-Derivat und/oder ein Agarose-Derivat umfasst; und
wobei das Primerset derart bestimmt ist, dass die Nukleotidsequenz eines Oligonukleotids, das als eine Sonde dient, in der Mitte einer zu vervielfältigenden Sequenz eingeschlossen ist.

2. Verfahren gemäß Anspruch 1, wobei das Gel, auf dem die Sonde immobilisiert ist, in einem Well oder Durchgangsloch (through-hole) in einem Substrat gehalten wird.

## Revendications

1. Procédé de détection d'un acide nucléique, qui comprend :
(a) une étape dans laquelle un gel, sur lequel une sonde d'acide nucléique est immobilisée, est mis en contact avec une solution réactionnelle qui contient un acide nucléique qui sert de matrice pour l'amplification d'acide nucléique, un ensemble d'amorces pour l'amplification d'acide nucléique, une unité nucléotidique et une enzyme d'extension d'ADN ;
(b) une étape dans laquelle le gel et la solution réactionnelle sont soumis à un cycle thermique pour effectuer une réaction d'amplification d'acide nucléique ;
(c) une étape dans laquelle des fragments d'acide nucléique ayant une longueur de base spécifique sont choisis parmi les fragments d'acide nucléique amplifié, dans lequel le choix des fragments d'acide nucléique ayant la longueur de base spécifique survient en utilisant un effet de tamis moléculaire des gels à sonde immobilisée ; et
(d) une étape dans laquelle les fragments d'acide nucléique choisis sont détectés ;
dans lequel le rapport (V/S) du volume du gel (V (µm³)) sur la superficie de contact du gel sur lequel la sonde est immobilisée et de la solution réactionnelle (S (µm²)) est de 50 ou plus ;
dans lequel la concentration du gel sur lequel la sonde est immobilisée est supérieure à 2 % en masse et inférieure à 5 % en masse ;
dans lequel le gel sur lequel la sonde est immobilisée comprend un dérivé de (méth)acrylamide substitué et/ou un dérivé d'agarose ; et
dans lequel l'ensemble d'amorces est déterminé de telle façon que la séquence nucléotidique d'un oligonucléotide qui sert de sonde est incluse au milieu d'une séquence à amplifier.

2. Procédé selon la revendication 1, dans lequel le gel sur lequel la sonde est immobilisée est maintenu dans un puits ou à travers un trou dans un substrat.


### SEQUENCE LISTING

<110> Mitsubishi Rayon Co., Ltd.
<120> A method of detecting a nucleic acid
<130> PCT12-0002
<150> JP 2011-027588
   <151> 2011-02-10
<160> 10
<170> PatentIn version 3.4
<210> 1
   <211> 24
   <212> DNA
   <213> Artificial
<220>
   <223> Synthetic DNA
<400> 1
   aggakgttgg cttagaagca gcca 24
<210> 2
   <211> 29
   <212> DNA
   <213> Artificial
<220>
   <223> Synthetic DNA
<400> 2
   gtattaagtg gaaaaggatg tggagttgc 29
<210> 3
   <211> 24
   <212> DNA
   <213> Artificial
<220>
   <223> Synthetic DNA
<400> 3
   ccggtacatt ttcggcgcag agtc 24
<210> 4
   <211> 29
   <212> DNA
   <213> Artificial
<220>
   <223> Synthetic DNA
<400> 4
   aactccgaat gccaatgact tatccttag 29
<210> 5
   <211> 25
   <212> DNA
   <213> Artificial
<220>
   <223> Synthetic DNA
<400> 5
   agccttcctc aggaaacctt aggca 25
<210> 6
   <211> 29
   <212> DNA
   <213> Artificial
<220>
   <223> Synthetic DNA
<400> 6
   aactccgaat gccaatgact tatccttag 29
<210> 7
   <211> 28
   <212> DNA
   <213> Artificial
<220>
   <223> Synthetic DNA
<400> 7
   ttcactgcgg ctttccgtta agaaagca 28
<210> 8
   <211> 29
   <212> DNA
   <213> Artificial
<220>
   <223> Synthetic DNA
<400> 8
   aactccgaat gccaatgact tatccttag 29
<210> 9
   <211> 27
   <212> DNA
   <213> Artificial
<220>
   <223> Synthetic DNA
<400> 9
   ccgcctatcc tgtacaaact gtaccaa 27
<210> 10
   <211> 2770
   <212> DNA
   <213> Bacillus cereus
<400> 10
   cacggtggat gccttgacac taggagtcga tgaaggacgg gactaacgcc gatatgcttc      60
   ggggagctgt aagtaagctt tgatccgaag atttccgaat ggggaaaccc actatacgta     120
   atggtatggt atccttacct gaatacatag ggtatggaag acagacccag ggaactgaaa     180
   catctaagta cctggaggaa gagaaagcaa atgcgatttc ctgagtagcg gcgagcgaaa     240
   cggaatctag cccaaaccaa gaggcttgcc tcttggggtt gtaggacatt ctatacggag     300
   ttacaaagga acgaggtaga cgaagcgacc tggaaaggtc cgtcgtagag ggtaacaacc     360
   ccgtagtcga aacttcgttc tctcttgaat gtatcctgag tacggcggaa cacgtgaaat     420
   tccgtcggaa tctgggagga ccatctccca aggctaaata ctccctagtg atcgatagtg     480
   aaccagtacc gtgagggaaa ggtgaaaagc accccggaag gggagtgaaa gagatcctga     540
   aaccgtgtgc ctacaaatag tcagagcccg ttaatgggtg atggcgtgcc ttttgtagaa     600
   tgaaccggcg agttacgatc ccgtgcaagg ttaagttgaa gagacggagc cgcagcgaaa     660
   gcgagtctga atagggcgtt tagtacgtgg tcgtagaccc gaaaccaggt gatctaccca     720
   tgtccagggt gaagttcagg taacactgaa tggaggcccg aacccacgca cgttgaaaag     780
   tgcggggatg aggtgtgggt agcggagaaa ttccaatcga acctggagat agctggttct     840
   ccccgaaata gctttagggc tagccttaag tgtaagagtc ttggaggtag agcactgatt     900
   gaactagggg tcctcatcgg attaccgaat tcagtcaaac tccgaatgcc aatgacttat     960
   ccttaggagt cagactgcga gtgataagat ccgtagtcaa gagggaaaca gcccagatcg    1020
   ccagctaagg tcccaaagtg tgtattaagt ggaaaaggat gtggagttgc ttagacaact    1080
   aggatgttgg cttagaagca gccaccattt aaagagtgcg taatagctca ctagtcgagt    1140
   gactctgcgc cgaaaatgta ccggggctaa atacaccacc gaagctgcga attgatacca    1200
   atggtatcag tggtagggga gcgttctaag tgcagtgaag tcagaccgga aggactggtg    1260
   gagcgcttag aagtgagaat gccggtatga gtagcgaaag acgggtgaga atcccgtcca    1320
   ccgaatgcct aaggtttcct gaggaaggct cgtccgctca gggttagtca ggacctaagc    1380
   cgaggccgac aggcgtaggc gatggacaac aggttgatat tcctgtacca cctctttatc    1440
   gtttgagcaa tggagggacg cagaaggata gaagaagcgt gcgattggtt gtgcacgtcc    1500
   aagcagttag gctgataagt aggcaaatcc gcttatcgtg aaggctgagc tgtgatgggg    1560
   aagctcctta tggagcgaag tctttgattc cccgctgcca agaaaagctt ctagcgagat    1620
   aaaaggtgcc tgtaccgcaa accgacacag gtaggcgagg agagaatcct aaggtgtgcg    1680
   agagaactct ggttaaggaa ctcggcaaaa tgaccccgta acttcgggag aaggggtgct    1740
   ttcttaacgg aaagccgcag tgaataggcc caagcgactg tttagcaaaa acacaggtct    1800
   ctgcgaagcc gtaaggcgaa gtataggggc tgacacctgc ccggtgctgg aaggttaagg   1860
   agaggggtta gcgtaagcga agctctgaac tgaagcccca gtaaacggcg gccgtaacta   1920
   taacggtcct aaggtagcga aattccttgt cgggtaagtt ccgacccgca cgaaaggtgt   1980
   aacgatttgg gcactgtctc aaccagagac tcggtgaaat tatagtacct gtgaagatgc   2040
   aggttacccg cgacaggacg gaaagacccc gtggagcttt actgtagcct gatattgaat   2100
   tttggtacag tttgtacagg ataggcggga gccattgaaa ccggagcgct agcttcggtg   2160
   gaggcgctgg tgggataccg ccctgactgt attgaaattc taacctacgg gtcttatcga   2220
   cccgggagac agtgtcaggt gggcagtttg actggggcgg tcgcctccta aagtgtaacg   2280
   gaggcgccca aaggttccct cagaatggtt ggaaatcatt cgtagagtgc aaaggcataa   2340
   gggagcttga ctgcgagacc tacaagtcga gcagggacga aagtcgggct tagtgatccg   2400
   gtggttccgc atggaagggc catcgctcaa cggataaaag ctaccccggg gataacaggc   2460
   ttatctcccc caagagtcca catcgacggg gaggtttggc acctcgatgt cggctcatcg   2520
   catcctgggg ctgtagtcgg tcccaagggt tgggctgttc gcccattaaa gcggtacgcg   2580
   agctgggttc agaacgtcgt gagacagttc ggtccctatc cgtcgtgggc gtaggaaatt   2640
   tgagaggagc tgtccttagt acgagaggac cgggatggac gcaccgctgg tgtaccagtt   2700
   gttctgccaa gggcatagct gggtagctat gtgcggaagg gataagtgct gaaagcatct   2760
   aagcatgaag                                                          2770